# EUROPÄISCHE PATENTSCHRIFT

(11) **EP 1 666 602 B1**
(45) Veröffentlichungstag und Bekanntmachung des Hinweises auf die Patenterteilung: **13.12.2006**
(21) Anmeldenummer: 04027280.9
(22) Anmeldetag: 17.11.2004
(51) Int. Cl.: C12P 21/00

(54) **Verfahren zum Herstellen eines heterologen Proteins unter Verwendung von Wirtszellen einer Hefeart**
Method for the preparation of a heterologous protein, using yeast cells
Méthode pour la préparation d'une protéine hétérologue, utilisant des cellules hôtes de levure

(43) Veröffentlichungstag der Anmeldung: 07.06.2006
(73) Patentinhaber: Artes Biotechnology GmbH, 45136 Essen (DE); RHEIN BIOTECH GESELLSCHAFT FÜR NEUE BIOTECHNOLOGISCHE PROZESSE UND PRODUKTE MBH, 40595 Düsseldorf (DE)
(72) Erfinder: Gellissen, Gerd, 42489 Wülfrath (DE); Klabunde, Jens, 40223 Düsseldorf (DE); Hollenberg, Cornelis, 40213 Düsseldorf (DE); Degelmann, Adelheid, 40597 Düsseldorf (DE)
(74) Vertreter: Schmidt, Frank-Michael

(56) Entgegenhaltungen:
- KLABUNDE: "Koproduktion pharmazeutischer Proteine und Hilfsfaktoren zur Optimierung mikrobieller Expressionssysteme bei Beschränkung auf ein einziges integratives Vektorsystem" [Online] 2003, UNIVERSITÄTS- UND LANDESBIBLIOTHEK DÜSSELDORF , DÜSSELDORF, DEUTSCHLAND , XP002328094 Gefunden im Internet: URL:http://diss.ub.uni-duesseldorf.de/ebib /diss/diss_files/672.pdf> [gefunden am 2005-05-12] Kapitel 3.2.1, 3.3.1, 3.4.1, 3.5.1, 4.3.1
- PUNT P J ET AL: "Filamentous fungi as cell factories for heterologous protein production" TRENDS IN BIOTECHNOLOGY, ELSEVIER PUBLICATIONS, CAMBRIDGE, GB, Bd. 20, Nr. 5, 1. Mai 2002 (2002-05-01), Seiten 200-206, XP004347070 ISSN: 0167-7799
- CONESA ANA ET AL: "Calnexin overexpression increases manganese peroxidase production in Aspergillus niger" APPLIED AND ENVIRONMENTAL MICROBIOLOGY, WASHINGTON,DC, US, Bd. 68, Nr. 2, Februar 2002 (2002-02), Seiten 846-851, XP002293059 ISSN: 0099-2240
- DE VIRGILIO CLAUDIO ET AL: "CNE1, a Saccharomyces cerevisiae homologue of the genes encoding mammalian calnexin and calreticulin" YEAST, Bd. 9, Nr. 2, 1993, Seiten 185-188, XP002328093 ISSN: 0749-503X
- HIGGINS M K ET AL: "Calnexin co-expression and the use of weaker promoters increase the expression of correctly assembled Shaker potassium channel in insect cells" BIOCHIMICA ET BIOPHYSICA ACTA. BIOMEMBRANES, AMSTERDAM, NL, Bd. 1610, Nr. 1, 17. Februar 2003 (2003-02-17), Seiten 124-132, XP004409763 ISSN: 0005-2736

## Beschreibung

Die Erfindung bezieht sich auf ein Verfahren zum Herstellen eines heterologen Proteins, bei dem Wirtszellen einer Hefeart bereitgestellt werden, die jeweils wenigstens eine für das heterologe Protein codierende rekombinante DNA-Sequenz enthalten, dann die Wirtszellen zu einer Expression und Sekretion des heterologen Proteins veranlaßt werden und das sezernierte, heterologe Protein abgetrennt wird.

Verfahren der eingangs genannten Art sind beispielsweise aus der Veröffentlichung WO 00/68400 A1 bekannt. Die Gewinnung von Proteinen, die beispielsweise als aktive Verbindungen in Medikamenten verwendet werden, mit Hilfe rekombinanter DNA-Technologien hat unter anderem den Vorteil, daß die Proteine in gut charakterisierten Wirten in praktisch unbegrenzter Menge zur Verfügung gestellt werden können. Für die Gewinnung rekombinanter Proteine ist es von Vorteil, wenn das jeweils exprimierte Protein sezerniert wird, so daß es anschließend aus dem Zellüberstand gewonnen werden kann. Dies vereinfacht die Aufbereitung in hohem Maße, da das Protein bereits in relativ reiner Form vorliegt und aufwendige Reinigungsschritte vermieden werden können. Häufig werden Hefen verwendet, um sekretorische Proteine in großem Maßstab zu gewinnen. Diese haben den Vorteil, daß sie relativ einfach in Zellkulturen gehalten werden können und zu einer guten Ausbeute führen.

Man ist stets bestrebt, die Sekretion des heterologen Proteins zu steigern. Um dies zu erreichen, wurde beispielsweise vorgeschlagen, die Anzahl der Kopien der für das heterologe Protein codierenden rekombinanten DNA-Sequenzen zu erhöhen. Ferner wurde für eine Steigerung der Ausbeute der häufig in glykosylierter Form sezernierten Proteine von Arima et al. in "Enhanced secretion of hydrophobic peptide fused lysozyme by the introduction of N-glycosylation signal and the disruption of calnexin gene in Saccharomyces cerevisiae", FEBS Letters 440 (1998), Seiten 89 - 92, vorgeschlagen, dasjenige Gen der Hefezellen auszuschalten, welches für das Chaperon Calnexin codiert. Auf der Grundlage der Ergebnisse dieser Arbeit konnte erwartet werden, daß das Calnexin die Sekretion heterologer Proteine aus Hefezellen mindert. Zwar beschrieben Conesa et al. in "Calnexin Overexpression Increases Manganese Peroxidase Production in Aspergillus niger", in Applied and Environmental Microbiology, Februar 2002, Seiten 846 - 851, daß eine Überexpression von Calnexin die Produktion von Manganperoxidase erhöht; jedoch betraf dies einerseits filamentöse Pilzkulturen und andererseits wurde der positive Einfluß der Calnexin-Überexpression auf die Ausbeute mit einem Einfluß des Calnexins auf die Häm-Einbindung in die Manganperoxidase begründet.

Aufgabe der Erfindung ist es, ein Verfahren zur Verfügung zu stellen, bei dem die Sekretion eines heterologen Proteins bei Hefe-Wirtszellen gesteigert wird.

Diese Aufgabe wird erfindungsgemäß durch ein Verfahren mit den Merkmalen des Patentanspruchs 1 gelöst.

Bei dem erfinderischen Verfahren zum Herstellen eines Proteins werden zunächst Wirtszellen von wenigstens einer Hefeart der Gattungen Saccharomyces, Schizosaccharomyces, Kluyveromyces, Hansenula, Pichia, Arxula, Schwanniomyces, Candida oder Yarrowia bereitgestellt, die neben ihrer wenigstens einen eigenen, homologen, für wenigstens ein erstes Calnexin codierenden DNA-Sequenz jeweils wenigstens eine für das Protein codierende rekombinante DNA-Sequenz sowie wenigstens eine zusätzliche, für wenigstens ein zweites Calnexin codierende rekombinante DNA-Sequenz enthalten, wobei das Protein in der Wirtszelle ein heterologes sezernierbares Protein darstellt. Beispielsweise enthält die Wirtszelle eine homologe DNA-Sequenz, die ein erstes (eigenes) Calnexin codiert. Die Wirtszellen könnten aber auch mehrere eigene, homologe DNA-Sequenzen enthalten, die für ein Calnexin oder auch mehrere verschiedene Calnexine codieren. Die bereitzustellenden Wirtszellen enthalten erfindungsgemäß eine oder mehrere zusätzliche, rekombinante DNA-Sequenzen, die für wenigstens ein zweites Calnexin codieren. Die Begriffe "erstes" und "zweites" Calnexin dienen im Rahmen dieser Erfindungsbeschreibung zur Unterscheidung der durch bereits vorhandene, homologe DNA-Sequenzen codierten Calnexine einerseits von den durch zusätzliche, heterologe DNA-Sequenzen codierten Calnexinen anderseits, ohne daß durch diese Begriffe allein eine strukturelle Verschiedenheit impliziert werden soll. Die zusätzlichen, rekombinanten DNA-Sequenzen können mehrere verschiedene rekombinante DNA-Sequenzen umfassen, die für gleiche oder verschiedene (zweite) Calnexine codieren. Vorzugsweise sind es aber mehrere Kopien derselben rekombinanten DNA-Sequenz, die sämtlich für dasselbe zweite Calnexin codieren. Vorzugsweise ist das zweite Calnexin (für das die rekombinanten DNA-Sequenzen codieren) dem ersten Calnexin (für das die eigene, homologe DNA-Sequenz codiert) strukturell ähnlich oder gleich ("strukturell ähnlich" soll an dieser Stelle bedeuten, daß einerseits Codons, die dieselben Aminosäuren codieren, gegeneinander ausgetauscht sein können, und darüber hinaus, daß einzelne Codons gegen solche ausgetauscht sein können, die andere Aminosäuren codieren, soweit dadurch die Funktionen des Calnexins höchstens unwesentlich beeinflußt werden). Unter einem Calnexin soll allgemein ein Chaperon im endoplasmatischen Reticulum (ER) irgendeiner Zelle eines Organismus verstanden werden, das über eine Bindung an den Oligosaccharidanteil Glc1Man9-GlcNAc2 an der Faltung und Qualitätskontrolle neu entstehender Glykoproteine beteiligt ist, bis diese Proteine korrekt gefaltet oder - aufgrund falscher Faltung - einem Abbau zugeführt sind. Der Begriff "ein Calnexin" soll - solange nichts anderes ausgesagt ist - ein Calnexin eine Hefezelle (CNE) ebenso wie Calnexin (CNX) oder Calreticulin (CNR) einer Säugerzelle oder entsprechende Proteine einer anderen Zelle umfassen.

Die Wirtszellen werden mit Hilfe der wenigstens einen für das Protein codierenden Sequenz zu einer Expression des heterologen Proteins und mit Hilfe der wenigstens einen zusätzlichen, für das wenigstens eine zweite Calnexin codierenden Sequenz zu einer Überexpression von Calnexinen veranlaßt. Anschließend werden die Wirtszellen zu einer Sekretion des heterologen Proteins veranlaßt. Schließlich wird das sezernierte, heterologe Protein abgetrennt.

Angesichts des oben genannten Standes der Technik ist die steigernde Wirkung der Überexpression von Calnexinen auf die Sekretion heterologer Proteine bei Hefezellen überraschend. Überraschend ist auch, daß eine weitere Steigerung der Protein-Sekretion durch Calnexin-Überexpression sogar dann festgestellt wurde, wenn die Wirtszellen bereits ohne Calnexin-Überexpression hohe Ausbeuten des sezernierten heterologen Proteins zeigten.

Bei der bevorzugten Ausführungsform des erfindungsgemäßen Verfahrens werden beim Schritt des Bereitstellens der Wirtszellen die wenigstens eine für das hetorologe Protein codierende Sequenz und die wenigstens eine zusätzliche, für wenigstens ein zweites Calnexin codierende DNA-Sequenz mit Hilfe von Vektoren in die Wirtszellen eingebracht. Die Vektoren könnten hierbei als freie Plasmide in der Zelle vorliegen. Vorzugsweise werden die jeweiligen Vektoren jedoch in das Genom der Zelle integriert.

Die für das hetorologe Protein codierenden rekombinanten DNA-Sequenzen einerseits und die zusätzlichen, für das wenigstens eine zweite Calnexin codierenden rekombinanten DNA-Sequenzen andererseits könnten auf einem gemeinsamen Vektor liegen. Vorzugsweise wird jedoch jede für das hetorologe Protein codierende DNA-Sequenz und jede für ein zweites Calnexin codierende DNA-Sequenz in jeweils einen separaten Vektor eingebracht. Dies vereinfacht die Bereitstellung der Vektoren.

Die Wirtszellen können auf verschiedene Weise bereitgestellt werden. Beispielsweise können zunächst rekombinante Wirtszellen bereitgestellt werden, die jeweils wenigstens eine das heterologe Portein codierende rekombinante DNA-Sequenz enthalten und anschließend die rekombinanten Wirtszellen supertransformiert werden, indem in die rekombinanten Wirtszellen jeweils die wenigstens eine zusätzliche, für wenigstens ein zweites Calnexin codierende DNA-Sequenz mit Hilfe von Vektoren eingebracht wird. Alternativ könnten die Wirtszellen bereitgestellt werden, indem zunächst rekombinante Wirtszellen bereitgestellt werden, die jeweils wenigstens eine zusätzliche, für wenigstens ein zweites Calnexin codierende DNA-Sequenz enthalten, und anschließend die rekombinanten Wirtszellen supertransformiert werden, indem in die rekombinanten Wirtszellen jeweils wenigstens eine für das heterologe Protein codierende rekombinante DNA-Sequenz eingebracht wird. Schließlich ist es auch denkbar, daß beim Schritt des Bereitstellens der Wirtszellen sowohl die wenigstens eine für das heterologe Protein codierende rekombinante DNA-Sequenz als auch die wenigstens eine für das wenigstens eine zweite Calnexin codierende rekombinante DNA-Sequenz gleichzeitig in die Wirtszelle eingebracht werden. Bei allen drei genannten Varianten werden vorzugsweise vor dem Schritt des Bereitstellens der Wirtszellen geeignete Expressionsplasmide als Vektoren bereitgestellt, die jeweils eine für das wenigstens eine zweite Calnexin codierende DNA-Sequenz bzw. eine für das heterologe Protein codierende DNA-Sequenz enthalten. Die letztgenannten Expressionsplasmide werden bereitgestellt, indem ein Calnexin-Gen (beispielsweise aus der Hefeart der Wirtszellen) isoliert und in ein Plasmid kloniert wird, wobei das Calnexin-Gen zwischen einen homologen oder heterologen Promotor und einen homologen oder heterologen Terminator eingefügt wird.

Bei einer bevorzugten Ausführungsform des erfindungsgemäßen Verfahrens werden im Schritt a) die Wirtszellen einer methylotrophen Hefe, insbesondere einer Hefe der Gattungen Hansenula und Pichia bereitgestellt. Besonders bevorzugt sind Wirtszellen der Hefe Hansenula polymorpha.
Bei einer bevorzugten Ausführungsform werden Wirtszellen einer rekombinanten Hefe bereitgestellt, die jeweils wenigstens eine

für ein heterologes Protein codierende rekombinante DNA-Sequenz enthalten, wobei das heterologe Protein ein Protein ist, das während der Expression und/oder Sekretion posttranslational modifiziert, insbesondere posttranslational glykosyliert wird. Dabei ist das Protein beispielsweise ein Protein aus einer Gruppe, die γ-Interferon, Alginatepimerase und Consensus-Phytase umfaßt.

Bei einer anderen Ausführungsform ist das heterologe Protein vorzugsweise ein Protein aus einer Gruppe, die γ-Interferon, α-Interferon, Hirudin, Serum Albumins, Alginatepimerase und Consensus-Phytase umfaßt.

Vorteilhafte und/oder bevorzugte Weiterbildungen des erfindungsgemäßen Verfahrens sind in den Unteransprüchen gekennzeichnet.

Im folgenden wird die Erfindung anhand bevorzugter Ausführungsformen näher beschrieben. In den Zeichnungen zeigen:
Fig. 1A und 1B: in die Hefe-Wirtszellen einbringbare zusätzliche für Calnexin codierende DNA-Sequenzen;
Fig. 2: eine Plasmidkarte des zur Supertransformation einer rekombinanten Hefe-Wirtszelle verwendeten Calnexin-Expressionsplasmids HCNE-Phleo;
Fig. 3: eine Plasmidkarte des zur Supertransformation einer rekombinanten Hefe-Wirtszelle verwendeten Calnexin-Expressionsplasmids TEFP-HCNE-Phleo; und
Fig. 4: einen Western-Blot eines Kulturüberstands eines Stamms von Hefezellen, die erfindungsgemäß zur Sekretion von Alginat-C5-Epimerase veranlaßt worden sind.

Zunächst werden Wirtszellen einer Hefeart bereitgestellt, die neben ihrer eigenen, homologen, für Calnexin codierenden DNA-Sequenz mehrere Kopien einer für ein heterologes Protein codierenden rekombinanten DNA-Sequenz sowie mehrere Kopien einer zusätzlichen, für Calnexin codierenden rekombinanten DNA-Sequenz enthalten. Vorzugsweise werden rekombinante Hansenula polymorpha-Wirtszellen bereitgestellt.

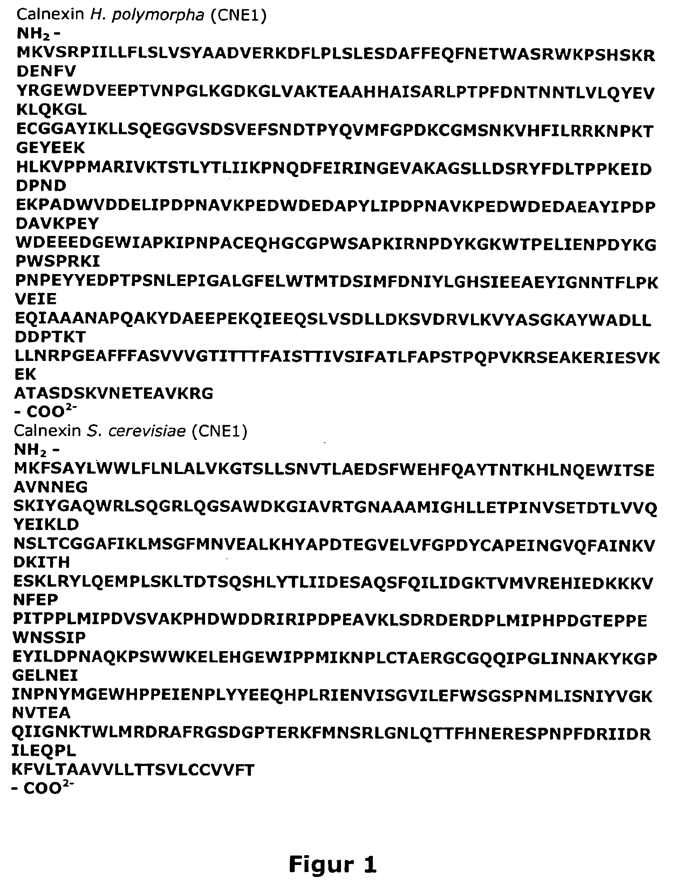

Zur Herstellung der rekombinanten Wirtszellen werden zunächst Expressionsplasmide als Vektoren hergestellt. Dazu wird das in Fig. 1A dargestellte Calnexin-Gen der Hefe Hansenula polymorpha (das Homologe des Calnexin-Gens CNE1 der Saccharomyces cerevisiae - vgl. Fig. 1B) aus einem Hansenula polymorpha-Stamm mit dem Namen RB11 durch Polymerasekettenreaktion isoliert, kloniert und sequenziert.

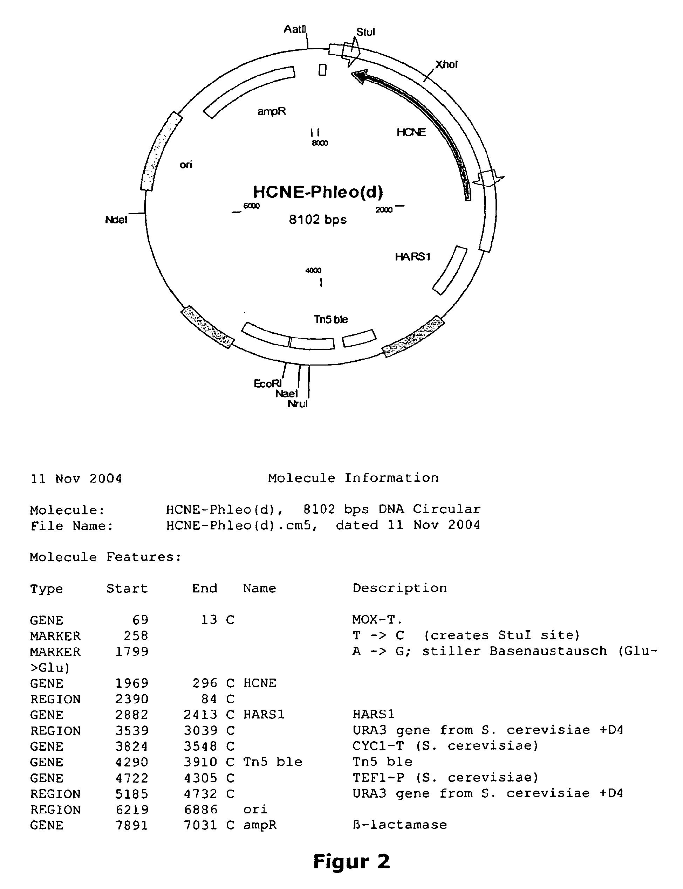

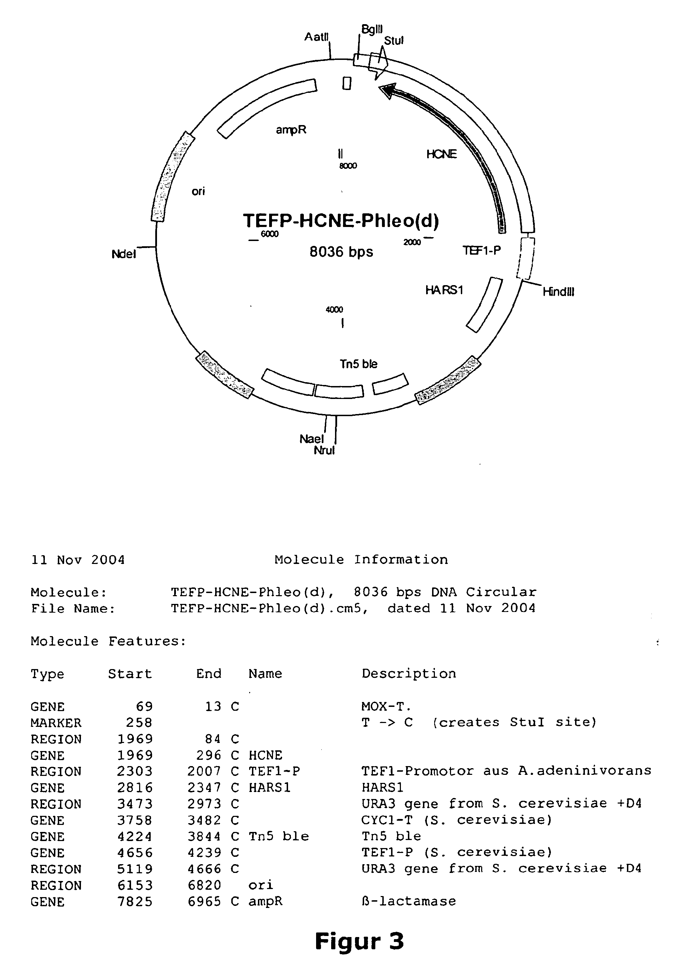

In Experimenten wurden zwei Expressionsplasmide verwendet, die das Calnexin-Gen der Hansenula polymorpha enthielten. In einem Expressionsplasmid mit dem Namen HCNE-Phleo ("HCNE" abgeleitet von: Hansenula Calnexin; "Phleo" abgeleitet vom Resistenzgen gegen das Selektions-Antibiotikum Phleomycin) wurde das Calnexin-Gen durch seinen ursprünglichen Transkriptionspromotor und seinen ursprünglichen Terminator flankiert. Dieses Plasmid ist in Fig. 2 veranschaulicht. Bei dem in Fig. 3 verschaulichten Expressionsplasmid TEFP-HCNE-Phleo ("TEFP" abgeleitet vom Transkriptionselongationsfaktor-Promotor) wurde das Calnexin-Gen durch den TEF1-Promotor aus der Hefe Arxula adeninivorans und den MOX-Terminator aus Hansenula polymorpha kontrolliert. Der in diesem Vektor verwendete TEF-Promotor für die Kontrolle der CNE-Expression kann als universelles Element für die Expression in verschiedenen Hefearten eingesetzt werden (vgl. Klabunde, J., Kunze, G., Gellissen, G. und Hollenberg, C.P., "Integration of heterologons genes in several yeast species using vectors containing a Hansenula polymorpha-derived DNA targeting", FEMS Yeast Research 4, 185-198, 2003). Die beiden Expressionsplasmide wurden anschließend zur Supertransformation dreier verschiedener rekombinanter Stämme der Hansenula polymorpha, die jeweils ein heterologes Protein sezernierten, verwendet. Von den drei untersuchten rekombinanten Proteinen war es bekannt, daß diese durch eine Glykosylierung posttranslational in den Wirtszellen der Hansenula polymorpha modifiziert wurden. Für die Supertransformation wurden die folgenden rekombinanten Stämme der Hansenula polymorpha ausgewählt:
a) RB11/AlgElsyn 52-4, der eine bakterielle Alginat-C5-Epimirase sezerniert;
b) RB11/Conphys 3-68, der eine synthetische "Consensus"-Phytase sezerniert; und
c) RB11/FMD-MFIFND 23-2, der eine Variante menschlichen γ-Interferons sezerniert, welches bei Hansenula polymorpha im hohen Maße überglykosyliert ist.

Jeder dieser Stämme wurde mit einem der beiden oben genannten Expressionsplasmide (HCNE-Phleo oder TEFP-HCNE-Phleo) supertransformiert. Die Supertransformanden wurden bezüglich der Resistenz gegen das Antibiotikum Phleomycin selektiert. Nach einer Stabilisierung wurden die Supertransformanden unter den Bedingungen der heterologen Proteinexpression kultiviert, und die sezernierten heterologen Proteine wurden durch Western-Blotting oder SDS-PAGE/Coomassie-Färbung analysiert.

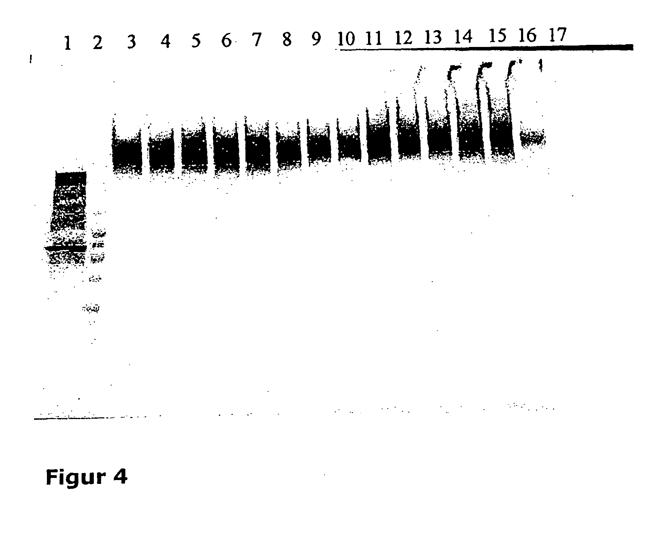

Fig. 4 zeigt beispielhaft einen Western-Blot von Kulturüberständen rekombinanter Hansenula polymorpha-Stämme RB11/AlgE1syn 52-4, die bakterielle Alginat-C5-Epimerase exprimieren und mit den Expressionsplasmiden HCNE-Phleo(d) (Spuren 3-8) oder TEFP-HCNE (Spuren 9-15) supertransformiert wurden. Spur 1 zeigt einen AlgE1-Standard (E.coli), Spur 2 Proteingrößenmarker. Spur 16 zeigt zum Vergleich einen nicht supertransformierten rekombinanten Stamm RB11/AlgE1syn 52-4 und Spur 17 einen Kontrollstamm, der nicht rekombinant ist.Die Ergebnisse dieser Analyse, insbesondere Fig. 4, zeigen deutlich eine erhöhte Menge sezernierten Proteins bei den analysierten Supertransformanden. Dies gilt für beide verwendete Calnexin-Expressionsplasmide.

Die Kopienzahl der Expressionsvektoren in den drei untersuchten rekombinanten Stämmen war unverändert, so daß die Erhöhung der Proteinmenge nicht auf einen Gendosiseffekt, sondern ausschließlich auf die Calnexinwirkung zurückzuführen ist. Die positive Wirkung der Calnexin-Überexpression war sowohl bei Stämmen mit geringer Zahl der integrierten Fremdgenvektoren (2 Kopien pro Zelle; RB11/AlgE1syn 52-4), als auch bei Stämmen mit hoher Zahl der integrierten Fremdgenvektoren (ca. 50 Kopien pro Zelle; RB11/FMD-MFIFNG 23-2 und RB11/Conphys 3-68) nachweisbar. Die positive Wirkung der Calnexin-Überexpression war ebenfalls über den gesamten Bereich der Fremdproteinproduktion (wenige Milligramm IFNgamma pro Liter in RB11/FMD-MFIFNG 23-2; 1 - 2 Gramm Epimerase pro Liter in RB11/AlgE1syn 52-4; 13,5 Gramm Phytase pro Liter in RB11/Conphys 3-68) in Hefe nachweisbar.

Die Überexpression des Calnexins als Chaperon des endoplasmatischen Retikulums bei einem rekombinanten Stamm der Hansenula polymorpha, der ein glykosyliertes heterologes Protein exprimiert und sezerniert, führt somit zu einer signifikanten Verbesserung der Sekretionseffizienz und der Ausbeute dieses heterologen Proteins.

### Material und Methoden:

Die für die Konstruktion der Expressionsvektoren, sowie für die Transformation, Anzucht und Analyse der Hefezellen verwendeten Methoden und Medien, sowie die Basisvektoren und Hefestämme sind beschrieben in:
G. Gellissen (ed), *"Hansenula polymorpha -* biology and applications", Wiley-VCH, Weinheim 2002, insbesondere in den Kapiteln:
   A. Degelmann, "Methods" und
   M. Suckow, G. Gellissen, "The expression platform based on *Hansenula polymorpha* strain RB11 and its derivatives - history, status and perspectives".

## Patentansprüche

1. Verfahren zum Herstellen eines Proteins, wobei:
a) Wirtszellen von wenigstens einer Hefeart der Gattungen Saccharomyces, Schizosaccharomyces, Kluyveromyces, Hansenula, Pichia, Arxula, Schwanniomyces, Candida und Yarrowia bereitgestellt werden, die neben ihrer wenigstens einen eigenen, homologen, für wenigstens ein erstes Calnexin codierenden DNA-Sequenz jeweils wenigstens eine für das Protein codierende rekombinante DNA-Sequenz sowie wenigstens eine zusätzliche, für wenigstens ein zweites Calnexin codierende rekombinante DNA-Sequenz enthalten, wobei das Protein in der Wirtszelle ein heterologes sezernierbares Protein darstellt;
b) die Wirtszellen mit Hilfe der wenigstens einen für das Protein codierenden Sequenz zu einer Expression des heterologen Proteins und mit Hilfe der wenigstens einen zusätzlichen, für das wenigstens eine zweite Calnexin codierenden Sequenz zu einer Überexpression von Calnexinen veranlaßt werden;
c) die Wirtszellen zu einer Sekretion des heterologen Proteins veranlaßt werden; und
d) das sezernierte, heterologe Protein abgetrennt wird.

2. Verfahren nach Anspruch 1, **dadurch gekennzeichnet, daß** im Schritt a) Wirtszellen bereitgestellt werden, die mehrere zusätzliche, für ein zweites Calnexin codierende rekombinante DNA-Sequenzen enthalten.

3. Verfahren nach Anspruch 2, **dadurch gekennzeichnet, daß** die mehreren zusätzlichen rekombinanten DNA-Sequenzen einander gleich sind.

4. Verfahren nach Anspruch 3, **dadurch gekennzeichnet, daß** das erste und das zweite Calnexin einander gleich sind.

5. Verfahren nach Anspruch 4, **dadurch gekennzeichnet, daß** die zusätzlichen, für Calnexin codierenden rekombinanten DNA-Sequenzen gleich einer der homologen, für Calnexin codierenden DNA-Sequenzen sind.

6. Verfahren nach einem der Ansprüche 1 - 5, **dadurch gekennzeichnet, daß** sämtliche für das heterologe Protein codierenden rekombinanten DNA-Sequenzen gleich sind.

7. Verfahren nach einem der Ansprüche 1 - 6, **dadurch gekennzeichnet, daß** beim Schritt des Bereitstellens der Wirtszellen die wenigstens eine für das heterologe Protein codierende DNA-Sequenz und die wenigstens eine zusätzliche für wenigstens ein zweites Calnexin codierende DNA-Sequenz mit Hilfe von Vektoren in die Wirtszellen eingebracht werden.

8. Verfahren nach Anspruch 7, **dadurch gekennzeichnet, daß** die Vektoren in das Genom der Zelle integriert werden.

9. Verfahren nach Anspruch 7 oder 8, **dadurch gekennzeichnet, daß** jede für das heterologe Protein codierende DNA-Sequenz und jede für ein zweites Calnexin codierende DNA-Sequenz in jeweils einen Vektor eingebracht wird.

10. Verfahren nach einem der Ansprüche 7 - 9, **dadurch gekennzeichnet, daß** als Vektoren Plasmide eingesetzt werden.

11. Verfahren nach einem der Ansprüche 1 - 10, **dadurch gekennzeichnet, daß** beim Schritt des Bereitstellens der Wirtszellen
a1) zunächst rekombinante Wirtszellen bereitgestellt werden, die jeweils wenigstens eine das heterologe Protein codierende rekombinante DNA-Sequenz enthalten, und
a2) anschließend die rekombinanten Wirtszellen supertransformiert werden, indem in die rekombinante Wirtszellen jeweils die wenigstens eine zusätzliche, für wenigstens ein zweites Calnexin codierende DNA-Sequenz mit Hilfe von Vektoren eingebracht wird.

12. Verfahren nach einem der Ansprüche 1 - 10, **dadurch gekennzeichnet, daß** beim Schritt des Bereitstellens der Wirtszellen
a1) zunächst rekombinante Wirtszellen bereitgestellt werden, die jeweils wenigstens eine zusätzliche, für wenigstens ein zweites Calnexin codierende DNA-Sequenz enthalten, und
a2) anschließend die rekombinanten Wirtszellen supertransformiert werden, indem in die rekombinanten Wirtszellen jeweils wenigstens eine für das heterologe Protein codierende rekombinante DNA-Sequenz eingebracht wird.

13. Verfahren nach einem der Ansprüche 1 - 10, **dadurch gekennzeichnet, daß** beim Schritt des Bereitstellens der Wirtszellen sowohl die wenigstens eine für das heterologe Protein codierende rekombinante DNA-Sequenz als auch die wenigstens eine für das wenigstens eine zweite Calnexin codierende rekombinante DNA-Sequenz gleichzeitig in die Wirtszelle eingebracht werden.

14. Verfahren nach einem der Ansprüche 11 - 13, **dadurch gekennzeichnet, daß** vor dem Schritt a) Expressionsplasmide als Vektoren bereitgestellt werden, die eine für das wenigstens eine zweite Calnexin codierende DNA-Sequenz bzw. eine für das heterologe Protein codierende DNA-Sequenz enthalten.

15. Verfahren nach Anspruch 14, **dadurch gekennzeichnet, daß** die Expressionsplasmide bereitgestellt werden, indem ein Calnexin-Gen isoliert und in ein Plasmid kloniert wird, wobei das Calnexin-Gen zwischen einem homologen oder heterologen Promotor und einem homologen oder heterologen Terminator eingefügt wird.

16. Verfahren nach einem der Ansprüche 1 - 15, **dadurch gekennzeichnet, daß** im Schritt a) Wirtszellen einer methylothrophen Hefe, insbesondere der Gattungen Hansenula und Pichia bereitgestellt werden.

17. Verfahren nach Anspruch 16, **dadurch gekennzeichnet, daß** im Schritt a) Wirtszellen der Hefe Hansenula polymorpha bereitgestellt werden.

18. Verfahren nach einem der Ansprüche 1 - 17, **dadurch gekennzeichnet, daß** im Schritt a) Wirtszellen einer rekombinanten Hefe bereitgestellt werden, die jeweils wenigstens eine für ein heterologes Protein codierende rekombinante DNA-Sequenz enthalten, wobei das heterologe Protein ein Protein ist, das während der Schritte b) und/oder c) posttranslational modifiziert wird.

19. Verfahren nach Anspruch 18, **dadurch gekennzeichnet, daß** das heterologe Protein ein Protein ist, das posttranslational glykosyliert wird.

20. Verfahren nach Anspruch 19, **dadurch gekennzeichnet, daß** das heterologe Protein ein Protein aus einer Gruppe ist, die γ-Interferon, Alginatepimerase und Consensus-Phytase und Varianten des γ-Interferons, der Alginatepimerase und der Consensus-Phytase umfaßt.

21. Verfahren nach einem der Ansprüche 1 - 17, **dadurch gekennzeichnet, daß** das heterologe Protein ein Protein aus einer Gruppe ist, die γ-Interferon, α-Interferon, Hirudin, Serum Albumin, Alginatepimerase, Consensus-Phytase und Varianten des γ-Interferons, α-Interferons, Hirudins, Serum Albumins, der Alginatepimerase und der Consensus-Phytase umfaßt.

## Claims

1. A process for producing a protein, where:
a) host cells are prepared from at least one yeast type of genera Saccharomyces, Schizosaccharomyces, Kluyveromyces, Hansenula, Pichia, Arxula, Schwanniomyces, Candida, and Yarrowia, which along with their at least one, own, homologous DNA sequence coding for at least a first calnexin, also contain in each case, at least one recombinant DNA sequence coding for the protein, as well as at least one additional recombinant DNA sequence coding for a second calnexin, where the protein in the host cell represents a heterologous, secretable protein;
b) the host cells are caused, with the help of at least one sequence coding for the protein, to express the heterologous proteins, and, with the help of at least one additional sequence coding for at least a second calnexin, to overexpress calnexins;
c) the host cells are caused to secrete the heterologous protein, and
d) the secreted heterologous protein is separated.

2. The process according to claim 1, **characterised in that** in step a), host cells are prepared that contain several additional recombinant DNA sequences coding for a second calnexin.

3. The process according to claim 2, **characterised in that** the several additional recombinant DNA sequences are identical to one another.

4. The process according to claim 3, **characterised in that** the first and the second calnexins are identical to each other.

5. The process according to claim 4, **characterised in that** the additional recombinant DNA sequences coding for calnexin are identical to one of the homologous DNA sequences coding for calnexin.

6. The process according to one of the claims 1-5, **characterised in that** all recombinant DNA sequences coding for the heterologous protein are identical.

7. The process according to the claims 1-6, **characterised in that** the step of preparing host cells, at least one DNA sequence coding for the heterologous protein and at least one additional DNA sequence coding for a second calnexin are inserted into the host cell with the aid of vectors.

8. The process according to claim 7, **characterised in that** the vectors are integrated into the genome of the cell.

9. THe process according to claim 7 or 8, **characterised in that** each DNA sequence coding for the heterologous protein and each DNA sequence coding for a second calnexin are each inserted into a vector.

10. The process according to one of the claims 7- 9, **characterised in that** plasmids are used as vectors.

11. The process according to one of claims 1-10, **characterised in that** during the step of preparing the host cells,
a1) recombinant host cells are first prepared that contain in each case at least one recombinant DNA sequence coding for a heterologous protein, and
a2) subsequently, the recombinant host cells are supertransformed by inserting into the recombinant host cells at least one additional DNA sequence coding in each case for at least a second calnexin, with the aid of vectors.

12. The process according to one of claims 1-10, **characterised in that** during the step of preparing the host cells,
a1) recombinant host cells are first prepared that contain in each case at least one additional recombinant DNA sequence coding for a second calnexin, and
a2) the recombinant host cells are subsequently supertransformed by inserting into the recombinant host cells at least one recombinant DNA sequence coding in each case for the heterologous protein.

13. The process according to the claims 1-10, **characterised in that** the step of preparing the host cells, at least one recombinant DNA sequence coding for the heterologous protein as well as at least one recombinant DNA sequence coding for at least a second calnexin are inserted into the host cell at the same time.

14. The process according to one of the claims 11-13, **characterised in that** before step a) expression plasmids are prepared as vectors that contain a DNA sequence for at least a second calnexin or a DNA sequence coding for the heterologous protein.

15. The process according to claim 14, **characterised in that** the expression plasmids are prepared, whereby a calnexin gene is isolated and cloned into a plasmid, where the calnexin gene is inserted between a homologous or heterologous promoter and a homologous or heterologous terminator.

16. The process according to one of the claims 1- 15, **characterised in that** in step a), host cells are prepared from a methylothrophenic yeast, in particular of the genera Hansenula and Pichia.

17. The process according to claim 16, **characterised in that** in step a), host cells are prepared from the yeast *Hansenula polymorpha.*

18. The process according to one of the claims 1-17, **characterised in that** in step a), host cells are prepared from a recombinant yeast, which in each case contain at least one recombinant DNA sequence coding for a heterologous protein, where the heterologous protein is a protein that is post-translationally modified during step b) and/or step c).

19. The process according to claim 18, **characterised in that** the heterologous protein is a protein that is post-translationally glycosylated.

20. The process according to claim 19, **characterised in that** the heterologous protein is a protein from a group that includes γ-interferon, alginate epimerase, and consensus phytase and variants of γ-interferon, alginate epimerase, and consensus phytase.

21. The process according to claims 1 to 17, **characterised in that** the heterologous protein is a protein from a group that includes γ-interferon, α-interferon, hirudin, serum albumin, alginate epimerase, consensus phytase, and variants of γ-interferon, α-interferon, hirudin, serum albumin, alginate epimerase, and consensus phytase.

## Revendications

1. Procédé pour la préparation d'une protéine, dans lequel:
a) des cellules hôtes d'au moins un type de levure des souches Saccharomyces, Schizosaccharomyces, Kluyveromyces, Hansenula, Pichia, Arxula, Schwanniomyces, Candida et Yarrowia sont préparées, qui contiennent, outre leur au moins une séquence d'ADN propre, homologue, codant pour au moins une première calnexine, dans chaque cas au moins une séquence d'ADN recombinante codant pour la protéine, ainsi qu'au moins une séquence d'ADN recombinante additionnelle, codant pour au moins une deuxième calnexine, la protéine dans la cellule hôte représentant une protéine sécrétable hétérologue;
b) les cellules hôtes sont induites à une expression de la protéine hétérologue au moyen de la au moins une séquence codant pour la protéine et à une surexpression des calnexines au moyen de la au moins une séquence additionnelle codant pour la au moins une deuxième calnexine;
c) les cellules hôtes sont induites à une sécrétion de la protéine hétérologue; et
d) la protéine hétérologue, sécrétée est séparée.

2. Procédé selon la revendication 1, **caractérisé par le fait que** dans l'étape a) on prépare des cellules hôtes qui contiennent plusieurs séquences d'ADN recombinantes additionnelles, codant pour une deuxième calnexine.

3. Procédé selon la revendication 2, **caractérisé par le fait que** les plusieurs séquences d'ADN recombinantes additionnelles sont identiques entre elles.

4. Procédé selon la revendication 3, **caractérisé par le fait que** la première et la deuxième calnexine sont identiques entre elles.

5. Procédé selon la revendication 4, **caractérisé par le fait que** les séquences d'ADN recombinantes additionnelles, codant pour la calnexine sont identiques à l'une des séquences d'ADN homologues, codant pour la calnexine.

6. Procédé selon l'une des revendications 1-5, **caractérisé par le fait que** toutes les séquences d'ADN recombinantes, codant pour la protéine hétérologue sont identiques.

7. Procédé selon l'une des revendications 1-6, **caractérisé par le fait que** dans l'étape de préparation des cellules hôtes la au moins une séquence d'ADN codant pour la protéine hétérologue et la au moins une séquence d'ADN additionnelle, codant pour au moins une deuxième calnexine sont introduites dans les cellules hôtes au moyen de vecteurs.

8. Procédé selon la revendication 7, **caractérisé par le fait que** les vecteurs sont intégrés dans le génome de la cellule.

9. Procédé selon la revendication 7 ou 8, **caractérisé par le fait que** chaque séquence d'ADN codant pour la protéine hétérologue et chaque séquence d'ADN codant pour une deuxième calnexine est introduite dans un vecteur à chaque fois.

10. Procédé selon l'une des revendications 7-9, **caractérisé par le fait qu'**on utilise comme vecteurs des plasmides.

11. Procédé selon l'une des revendications 1-10, **caractérisé par le fait que** dans l'étape de préparation des cellules hôtes
a1) on prépare d'abord des cellules hôtes recombinantes, qui contiennent à chaque fois au moins une séquence d'ADN recombinante codant pour la protéine hétérologue, et
a2) on supertransforme ensuite les cellules hôtes recombinantes, tandis que dans les cellules hôtes recombinantes à chaque fois la au moins une séquence d'ADN additionnelle, codant pour au moins une deuxième calnexine est introduite au moyen de vecteurs.

12. Procédé selon l'une des revendications 1-10, **caractérisé par le fait que** dans l'étape de préparation des cellules hôtes
a1) on prépare d'abord des cellules hôtes recombinantes qui contiennent à chaque fois au moins une séquence d'ADN additionnelle, codant pour au moins une deuxième calnexine, et
a2) on supertransforme ensuite les cellules hôtes recombinantes, tandis que dans les cellules hôtes recombinantes à chaque fois au moins une séquence d'ADN recombinante, codant pour la protéine hétérologue est introduite.

13. Procédé selon l'une des revendications 1-10, **caractérisé par le fait que**, dans l'étape de préparation des cellules hôtes, on introduit simultanément dans la cellule hôte aussi bien la au moins une séquence d'ADN recombinante codant pour la protéine hétérologue que la au moins une séquence d'ADN recombinante codant pour la au moins une deuxième calnexine.

14. Procédé selon l'une des revendications 11-13, **caractérisé par le fait que**, avant l'étape a) on prépare, à titre de vecteurs, des plasmides d'expression qui contiennent respectivement une séquence d'ADN codant pour la au moins une deuxième calnexine ou une séquence d'ADN codant pour la protéine hétérologue.

15. Procédé selon la revendication 14, **caractérisé par le fait qu'**on prépare les plasmides d'expression en isolant un gène de calnexine et en le clonant dans un plasmide, tandis que le gène de calnexine est inséré entre un promoteur homologue ou hétérologue et un terminateur homologue ou hétérologue.

16. Procédé selon l'une des revendications 1-15, **caractérisé par le fait que**, dans l'étape a), on prépare des cellules hôtes d'une levure méthylotrophe, en particulier des espèces Hansenula et Pichia.

17. Procédé selon la revendication 16, **caractérisé par le fait que**, dans l'étape a), on prépare des cellules hôtes de la levure Hansenula polymorpha.

18. Procédé selon l'une des revendications 1-17, **caractérisé par le fait que**, dans l'étape a), on prépare des cellules hôtes d'une levure recombinante, qui contiennent à chaque fois au moins une séquence d'ADN recombinante codant pour une protéine hétérologue, la protéine hétérologue étant une protéine qui est modifiée de manière post-traductionnelle pendant les étapes b) et/ ou c).

19. Procédé selon la revendication 18, **caractérisé par le fait que** la protéine hétérologue est une protéine qui est soumise à une glycosylation post-traductionnelle.

20. Procédé selon la revendication 19, **caractérisé par le fait que** la protéine hétérologue est une protéine choisie dans un groupe comprenant l'interféron γ, l'alginate-épimérase et la phytase de concensus et des variantes de l'interféron γ, de l'alginate-épimérase et de la phytase de concensus.

21. Procédé selon l'une des revendications 1-17, **caractérisé par le fait que** la protéine hétérologue est une protéine qui est choisie dans un groupe comprenant l'interféron γ, l'interféron α, l'hirudine, la sérum-albumine, l'alginate-épimérase, la phytase de consensus et des variantes de l'interféron y, de l'interféron α, de l'hirudine, de la sérum-albumine, de l'alginate-épimérase et de la phytase de consensus.


### SEQUENCE LISTING

<110> Artes Biotechnology GmbH
<120> Verfahren zum Herstellen eines heterologen Proteins unter Verwendung von Wirtszellen einer Hefeart
<130> A549EP
<140> 04 027 280.9
<141> 2004-11-17
<160> 2
<170> PatentIn version 3.1
<210> 1
<211> 557
<212> PRT
<213> Hansenula polymorpha
<400> 1
   Met Lys Val Ser Arg Pro Ile Ile Leu Leu Phe Leu Ser Leu Val Ser
   1               5                   10                  15
   Tyr Ala Ala Asp Val Glu Arg Lys Asp Phe Leu Pro Leu Ser Leu Glu
               20                  25                  30
   Ser Asp Ala Phe Phe Glu Gln Phe Asn Glu Thr Trp Ala Ser Arg Trp
           35                  40                  45
   Lys Pro Ser His Ser Lys Arg Asp Glu Asn Phe Val Tyr Arg Gly Glu
       50                  55                  60
   Trp Asp Val Glu Glu Pro Thr Val Asn Pro Gly Leu Lys Gly Asp Lys
   65                  70                  75                  80
   Gly Leu Val Ala Lys Thr Glu Ala Ala His His Ala Ile Ser Ala Arg
                   85                  90                  95
   Leu Pro Thr Pro Phe Asp Asn Thr Asn Asn Thr Leu Val Leu Gln Tyr
               100                 105                 110
   Glu Val Lys Leu Gln Lys Gly Leu Glu Cys Gly Gly Ala Tyr Ile Lys
           115                 120                 125
   Leu Leu Ser Gln Glu Gly Gly Val Ser Asp Ser Val Glu Phe Ser Asn
       130                 135                 140
   Asp Thr Pro Tyr Gln Val Met Phe Gly Pro Asp Lys Cys Gly Met Ser
   145                 150                 155                 160
   Asn Lys Val His Phe Ile Leu Arg Arg Lys Asn Pro Lys Thr Gly Glu
                   165                 170                 175
   Tyr Glu Glu Lys His Leu Lys Val Pro Pro Met Ala Arg Ile Val Lys
               180                 185                 190
   Thr Ser Thr Leu Tyr Thr Leu Ile Ile Lys Pro Asn Gln Asp Phe Glu
           195                 200                 205
   Ile Arg Ile Asn Gly Glu Val Ala Lys Ala Gly Ser Leu Leu Asp Ser
       210                 215                 220
   Arg Tyr Phe Asp Leu Thr Pro Pro Lys Glu Ile Asp Asp Pro Asn Asp
   225                 230                 235                 240
   Glu Lys Pro Ala Asp Trp Val Asp Asp Glu Leu Ile Pro Asp Pro Asn
                   245                 250                 255
   Ala Val Lys Pro Glu Asp Trp Asp Glu Asp Ala Pro Tyr Leu Ile Pro
               260                 265                 270
   Asp Pro Asn Ala Val Lys Pro Glu Asp Trp Asp Glu Asp Ala Glu Ala
           275                 280                 285
   Tyr Ile Pro Asp Pro Asp Ala Val Lys Pro Glu Tyr Trp Asp Glu Glu
       290                 295                 300
   Glu Asp Gly Glu Trp Ile Ala Pro Lys Ile Pro Asn Pro Ala Cys Glu
   305                 310                 315                 320
   Gln His Gly Cys Gly Pro Trp Ser Ala Pro Lys Ile Arg Asn Pro Asp
                   325                 330                 335
   Tyr Lys Gly Lys Trp Thr Pro Glu Leu Ile Glu Asn Pro Asp Tyr Lys
               340                 345                 350
   Gly Pro Trp Ser Pro Arg Lys Ile Pro Asn Pro Glu Tyr Tyr Glu Asp
           355                 360                 365
   Pro Thr Pro Ser Asn Leu Glu Pro Ile Gly Ala Leu Gly Phe Glu Leu
       370                 375                 380
   Trp Thr Met Thr Asp Ser Ile Met Phe Asp Asn Ile Tyr Leu Gly His
   385                 390                 395                 400
   Ser Ile Glu Glu Ala Glu Tyr Ile Gly Asn Asn Thr Phe Leu Pro Lys
                   405                 410                 415
   Val Glu Ile Glu Glu Gln Ile Ala Ala Ala Asn Ala Pro Gln Ala Lys
               420                 425                 430
   Tyr Asp Ala Glu Glu Pro Glu Lys Gln Ile Glu Glu Gln Ser Leu Val
           435                 440                 445
   Ser Asp Leu Leu Asp Lys Ser Val Asp Arg Val Leu Lys Val Tyr Ala
       450                 455                 460
   Ser Gly Lys Ala Tyr Trp Ala Asp Leu Leu Asp Asp Pro Thr Lys Thr
   465                 470                 475                 480
   Leu Leu Asn Arg Pro Gly Glu Ala Phe Phe Phe Ala Ser Val Val Val
                   485                 490                 495
   Gly Thr Ile Thr Thr Thr Phe Ala Ile Ser Thr Thr Ile Val Ser Ile
               500                 505                 510
   Phe Ala Thr Leu Phe Ala Pro Ser Thr Pro Gln Pro Val Lys Arg Ser
           515                 520                 525
   Glu Ala Lys Glu Arg Ile Glu Ser Val Lys Glu Lys Ala Thr Ala Ser
       530                 535                 540
   Asp Ser Lys Val Asn Glu Thr Glu Ala Val Lys Arg Gly
   545                 550                 555
<210> 2
<211> 502
<212> PRT
<213> Saccharomyces cerevisiae
<400> 2
   Met Lys Phe Ser Ala Tyr Leu Trp Trp Leu Phe Leu Asn Leu Ala Leu
   1               5                   10                  15
   Val Lys Gly Thr Ser Leu Leu Ser Asn Val Thr Leu Ala Glu Asp Ser
               20                  25                  30
   Phe Trp Glu His Phe Gln Ala Tyr Thr Asn Thr Lys His Leu Asn Gln
           35                  40                  45
   Glu Trp Ile Thr Ser Glu Ala Val Asn Asn Glu Gly Ser Lys Ile Tyr
       50                  55                  60
   Gly Ala Gln Trp Arg Leu Ser Gln Gly Arg Leu Gln Gly Ser Ala Trp
   65                  70                  75                  80
   Asp Lys Gly Ile Ala Val Arg Thr Gly Asn Ala Ala Ala Met Ile Gly
                   85                  90                  95
   His Leu Leu Glu Thr Pro Ile Asn Val Ser Glu Thr Asp Thr Leu Val
               100                 105                 110
   Val Gln Tyr Glu Ile Lys Leu Asp Asn Ser Leu Thr Cys Gly Gly Ala
           115                 120                 125
   Phe Ile Lys Leu Met Ser Gly Phe Met Asn Val Glu Ala Leu Lys His
       130                 135                 140
   Tyr Ala Pro Asp Thr Glu Gly Val Glu Leu Val Phe Gly Pro Asp Tyr
   145                 150                 155                 160
   Cys Ala Pro Glu Ile Asn Gly Val Gln Phe Ala Ile Asn Lys Val Asp
                   165                 170                 175
   Lys Ile Thr His Glu Ser Lys Leu Arg Tyr Leu Gln Glu Met Pro Leu
               180                 185                 190
   Ser Lys Leu Thr Asp Thr Ser Gln Ser His Leu Tyr Thr Leu Ile Ile
           195                 200                 205
   Asp Glu Ser Ala Gln Ser Phe Gln Ile Leu Ile Asp Gly Lys Thr Val
       210                 215                 220
   Met Val Arg Glu His Ile Glu Asp Lys Lys Lys Val Asn Phe Glu Pro
   225                 230                 235                 240
   Pro Ile Thr Pro Pro Leu Met Ile Pro Asp Val Ser Val Ala Lys Pro
                   245                 250                 255
   His Asp Trp Asp Asp Arg Ile Arg Ile Pro Asp Pro Glu Ala Val Lys
               260                 265                 270
   Leu Ser Asp Arg Asp Glu Arg Asp Pro Leu Met Ile Pro His Pro Asp
           275                 280                 285
   Gly Thr Glu Pro Pro Glu Trp Asn Ser Ser Ile Pro Glu Tyr Ile Leu
       290                 295                 300
   Asp Pro Asn Ala Gln Lys Pro Ser Trp Trp Lys Glu Leu Glu His Gly
   305                 310                 315                 320
   Glu Trp Ile Pro Pro Met Ile Lys Asn Pro Leu Cys Thr Ala Glu Arg
                   325                 330                 335
   Gly Cys Gly Gln Gln Ile Pro Gly Leu Ile Asn Asn Ala Lys Tyr Lys
               340                 345                 350
   Gly Pro Gly Glu Leu Asn Glu Ile Ile Asn Pro Asn Tyr Met Gly Glu
           355                 360                 365
   Trp His Pro Pro Glu Ile Glu Asn Pro Leu Tyr Tyr Glu Glu Gln His
       370                 375                 380
   Pro Leu Arg Ile Glu Asn Val Ile Ser Gly Val Ile Leu Glu Phe Trp
   385                 390                 395                 400
   Ser Gly Ser Pro Asn Met Leu Ile Ser Asn Ile Tyr Val Gly Lys Asn
                   405                 410                 415
   Val Thr Glu Ala Gln Ile Ile Gly Asn Lys Thr Trp Leu Met Arg Asp
               420                 425                 430
   Arg Ala Phe Arg Gly Ser Asp Gly Pro Thr Glu Arg Lys Phe Met Asn
           435                 440                 445
   Ser Arg Leu Gly Asn Leu Gln Thr Thr Phe His Asn Glu Arg Glu Ser
       450                 455                 460
   Pro Asn Pro Phe Asp Arg Ile Ile Asp Arg Ile Leu Glu Gln Pro Leu
   465                 470                 475                 480
   Lys Phe Val Leu Thr Ala Ala Val Val Leu Leu Thr Thr Ser Val Leu
                   485                 490                 495
   Cys Cys Val Val Phe Thr
               500